# EUROPÄISCHE PATENTSCHRIFT

(11) **EP 3 606 494 B1**
(45) Veröffentlichungstag und Bekanntmachung des Hinweises auf die Patenterteilung: **30.06.2021**
(21) Anmeldenummer: 18716976.8
(22) Anmeldetag: 03.04.2018
(51) Int. Cl.: A61Q 11/02, A61Q 11/00, A61K 8/60, A61K 8/02

(54) **PULVER ZUR REINIGUNG EINES INNEREN KÖRPERTEILS UND/ODER EINES IMPLANTATS, VERFAHREN ZUR HERSTELLUNG EINES SOLCHEN PULVERS UND GEEIGNETE VERWENDUNGEN**
POWDER FOR CLEANING AN INNER BODY PART AND / OR IMPLANT, METHOD FOR PRODUCING SUCH A POWDER, AND CORRESPONDING USES
POUDRE DE NETTOYAGE D'UNE PIECE DE CORPS INTERNE ET / OU D'UN IMPLANT, PROCEDE DE PRODUCTION D'UNE TELLE POUDRE ET UTILISATIONS APPROPRIEES

(30) Priorität: 03.04.2017 DE 102017107124
(43) Veröffentlichungstag der Anmeldung: 12.02.2020
(73) Patentinhaber: Ferton Holding S.A., 2800 Delémont (CH)
(72) Erfinder: DONNET, Marcel, 01630 Saint Jean de Gonville (FR)
(74) Vertreter: Müller Schupfner & Partner Patent- und Rechtsanwaltspartnerschaft mbB
(86) Internationale Anmeldenummer: PCT/EP2018/058481
(87) Internationale Veröffentlichungsnummer: WO 2018/134446

(56) Entgegenhaltungen:
- EP-A1- 2 228 175
- CN-A- 101 613 119
- DE-A1-102013 109 758
- US-A1- 2012 263 704

## Beschreibung

Die vorliegende Erfindung betrifft ein Pulver zur Reinigung eines inneren Körperteils und/oder eines Implantats gemäß Ansprüchen 1 - 5, ein Verfahren zur Herstellung eines solchen Pulvers gemäß Ansprüchen 6-11 und die Verwendung des Pulvers gemäß Anspruch 12.

Das Reinigen von inneren Körperteilen, wie z. B. Knochen, und Implantaten, z. B. im Vorfeld einer Operation, ist ein typisches Erfordernis, um beispielsweise prophylaktisch bakterielle Infektionen zu vermeiden. Dabei erweist sich das Reinigen von Implantaten allerdings gerade deswegen als Herausforderung, weil Implantate wegen ihren hohen Anforderungen an ihre Biokompatibilität und ihre Fähigkeit zum möglichst schnellen Verwachsen mit menschlichem bzw. tierischem Gewebe bzw. inneren Körperteilen, meist aus Materialien gefertigt bzw. mit Materialien beschichtet sind, deren Oberflächen anfällig sind für Beschädigungen. Typischerweise sind diese Materialien porös und hydrophil.

Aus der Zahnmedizin ist die Verwendung von Pulver-Luft-Gemischen bekannt, die zu Reinigungszwecken mittels Pulverstrahlgeräten auf die Zahnoberfläche aufgestrahlt werden. Allerdings würde der Einsatz eines solchen Pulver-Luft-Gemischs eine anschließende Sterilisation der inneren Körperteile bzw. Implantate erforderlich machen, bevor diese in den Körper eingesetzt werden können. Siehe insbesondere EP 2 228 175 A1 und DE 10 2013 109758 A1.

Es ist daher eine Aufgabe der vorliegenden Erfindung, eine Möglichkeit bereitzustellen, mit der innere Körperteile und/oder Implantate möglichst schonend gereinigt werden können und kein zwingend erforderlicher Nachbehandlungsschritt nötig ist.

Diese Aufgabe wird gelöst durch ein Pulver gemäß Anspruch 1 und ein Verfahren gemäß Anspruch 5. Weitere Vorteile und Merkmale der Erfindung ergeben sich aus den Unteransprüchen sowie der Beschreibung und den beigefügten Figuren.

Erfindungsgemäß ist ein Pulver zur Reinigung eines inneren Körperteils und/oder eines Implantats, insbesondere mittels eines Pulverstrahlgeräts, vorgesehen, wobei das Pulver infolge eines Sterilisierungsvorgangs steril, d. h. keim- und bakterienfrei bzw. im Wesentlichen frei von vermehrungsfähigen Mikroorganismen, ist. Gegenüber dem Stand der Technik lässt sich mit Vorteil erfindungsgemäß ein steriles Pulver bereitstellen, mit dem sich innere Körperteile und/oder Implantate, insbesondere eine Oberfläche der inneren Körperteile und/oder der Implantate, einfach abrasiv reinigen lassen und zwar auch in sterilen OP-Umgebungen. Durch den Sterilisierungsvorgang ist das Pulver in vorteilhafter Weise derart präpariert, dass nach der Reinigung kein zusätzlicher Nachbehandlungsschritt, insbesondere zeitintensive Sterilisierung, des inneren Körperteils und/oder Implantates erforderlich ist. Dies gestattet in vorteilhafter Weise auch eine kurzfristige bzw. partielle Reinigung unter Einsatz des Pulvers.

Unter dem Begriff Implantat sind insbesondere gewebefreie Implantate, wie z. B. Implantate zum Knochen- oder Zahnersatz, und unter inneren Körperteilen Knochen, wie z. B. Kieferknochen oder Hüftknochen, zu verstehen. Beispielsweise handelt es sich bei dem Implantat um ein Hüftknochenimplantat, ein Kieferknochenimplantat oder Vergleichbares.

Insbesondere ist unter dem Begriff Pulver ein jedes Pulver oder Pulvergemisch zu verstehen, das zur Verwendung in einem Pulverstrahlgerät geeignet ist, d. h. aus dem sich mittels des Pulverstrahlgeräts ein Pulver-Luft-Gemisch bilden lässt. Grundsätzlich fallen unter den Begriff Pulver jede Akkumulation von Teilchen bzw. Partikel. Dabei kann es sich auch um ein Gemisch aus Partikeln verschiedener Materialien bzw. Materialzusammensetzungen handeln. Weiterhin ist den Partikeln des Pulvers vorteilhafter Weise eine abrasive Eigenschaft zuzusprechen, d. h. die Partikel sind derart ausgestaltet bzw. ausgewählt, dass sie, wenn sie in einem Strahl eines Pulver-Luft-Gemisches auf eine Oberfläche des inneren Körperteils oder des Implantats treffen, diese Oberfläche abrasiv reinigen. Dabei ist es ein weiterer Vorteil, dass sich das Pulver Implantat spezifisch auswählen lässt, d. h. das Pulver kann derart ausgewählt werden, dass eine Wahrscheinlichkeit für eine Oberflächenschädigung für das jeweilige Implantat bei dessen Reinigung mit dem Pulver individuell reduziert werden kann. Vorzugsweise ist unter einem sterilen Pulver ein solches zu verstehen, dessen Restgehalt an vermehrungsfähigen Mikroorganismen im Pulver höchstens 10⁻⁶ koloniebildende Einheiten beträgt, d. h. in einer Million gleichbehandelten Partikeln des Pulvers darf maximal ein vermehrungsfähiger Mikroorganismus enthalten sein.

Insbesondere sind unter dem Ausdruck "im Wesentlichen" solche Abweichungen zu verstehen, die vom jeweiligen exakten Wert um +/- 15%, bevorzugt um +/-10%, besonders bevorzugt +/- 5% abweichen und/oder Abweichungen in Form von für die Funktion unbedeutenden Änderungen entsprechen.

Gemäß einer weiteren Ausführungsform der vorliegenden Erfindung ist es vorgesehen, dass das Pulver Natriumbikarbonat bzw. Natriumhydrogencarbonat (NaHCO₂), Kalziumkarbonat (CaCO₃), Aluminiumtrihydroxid (Al[OH]3), Kalzium-Natrium-Phosphosilikat (CaNaO₆PSi), Zuckeralkohole wie Erythritol (C₄H₁₀O₄), Disaccharide, insbesondere Trehalose (C₁₂H₂₂O₁₁) oder Isomaltuloseund/oder Glycin bzw. Glykokoll, Aminoessigsäure (C₂H₅NO₂), je nach Anwendungszweck, umfasst. Dabei handelt es sich in vorteilhafter Weise um Stoffzusammensetzungen, die bei der Oberflächenbehandlung von Zähnen bereits verwendet werden. Hierbei hat sich in überraschender Weise herausgestellt, dass sich diese Stoffzusammensetzungen zur Reinigung von Knochen und/oder Implantaten eignen. Besonders bevorzugt handelt es sich um Erythritol und/oder Glycin mit mittleren Korngrößen von ca. 10-20 µm, bevorzugt ca. 14 µm, die eine vergleichsweise schonende Behandlung aufgrund ihrer Beschaffenheit ermöglichen. Insbesondere handelt es sich bei dem Pulver um ein Pulver auf Natriumbikarbonat-Basis. Dabei weist das Pulver auf Natriumbikarbonat-Basis vorzugsweise eine mittlere Korngröße von im Wesentlichen 12 - 20 µm auf. Vorzugsweise ist es vorgesehen, dass das Pulver, insbesondere ein Natriumbikarbonat umfassendes Pulver, eine durchschnittliche Korngröße zwischen 5 und 100 µm, bevorzugt zwischen 10 und 50 µm und besonders bevorzugt im Wesentlichen von 17 µm aufweist. Insbesondere hat sich herausgestellt, dass sich Natriumbikarbonat vergleichsweise einfach sterilisieren lässt und zudem bei einer Reduzierung auf eine Korngröße von im Wesentlichen 17 µm, die Wahrscheinlichkeit für eine Schädigung durch das Pulver-Luft-Gemisch, das dieses Natriumbikarbonat umfasst, im Allgemeinen gemindert ist im Vergleich zu einem grobkörnigeren Natriumbikarbonatpulver. Im Fall der hier im Absatz angegebenen Zahlenwerte für die Korngrößen ist das Pulver vorzugsweise derart gestaltet, dass sich die Korngrößen mit einer Standardabweichung σ von 20 µm, vorzugsweise 10 µm und besonders bevorzugt von 5 µm um die durchschnittliche Korngröße verteilen. Erythritol-Pulver-Wasser-Gemische können unproblematisch am Zahnfleischrand, am Tascheneingang sowie auch gezielt innnerhalb der Tasche appliziert werden. Erythrit-Pulver eignet sich sowohl zur Entfernung von leichten Belägen und Verfärbungen, zur Zahnpolitur sowie zur Biofilmentfernung insbesondere und auf empfindlichen Implantatoberflächen.

In der vorliegenden Erfindung ist es vorgesehen, dass das Pulver eine Dichte zwischen 0,2 und 1,6 g/cm³ aufweist. Dabei hat es sich mit Vorteil herausgestellt, dass sich solche Pulver mit einer Dichte zwischen 0,2 und 1,6 g/cm³ einfach sterilisieren lassen und gleichzeitig beim Reinigen mittels eines Pulverstrahlgeräts selten Oberflächenschädigungen an inneren Körperteilen und/oder Implantaten verursachen.

Bevorzugt kommen bei der Pulverstrahlreiningung gemäß der Erfindung wasserlösliche Strahlmittel zum Einsatz, obwohl diese generell schwieriger zu sterilisieren sind. Nicht-wasserlösliche Substanzen hinterlassen nämlich bei der Anwendung beim Patienten, insbesondere im Mundraum, einen "typisch sandigen" Eindruck und sind auch nur schwer wieder zu entfernen. Das bezieht sich sowohl auf den Mundraum, als auch auf die Praxisräume des Arztes. Zudem sind Schädigungen oder der Verbleib von nicht wasserlöslichen Pulver-Teilchen in der Lunge zu befürchten, wenn diese nicht mittels eines Fluids, wie Wasser, gelöst und ausgeschwemmt warden können. Die wasserlösliche Eigenschaft ist daher für die erfindungsgemäßen Pulver vorteilhaft.

Als wasserlöslich werden gemäß der vorliegenden Erfindung Stoffe eingestuft, bei denen sich mehr als 1g Pulver pro Liter auflösen lässt. In einer weiteren Ausführungsform der vorliegenden Erfindung ist es daher vorgesehen, dass das Pulver wasserlöslich ist, wobei die Wasserlöslichkeit bei einer Temperatur von 20°C bei über 1 g/l, bevorzugt bei über 50 g/l, bevorzugt bei etwa 100 g/l, oder sogar bei über 100 g/l liegt. Die bevorzugten Pulver weisen dabei folgende Eigenschaften auf:

**(Tabelle 1)**

| Pulver | Löslichkeit in Wasser (g/l bei 20°C) | Dichte (g/cm³) |
|---|---|---|
| Erythritol | 540 | 1,45 |
| Trehalose | 690 | 1,80 |
| Glycin | 225 | 1,61 |
| Natriumbikarbonat | 96 | 2,54 |

Erythritol hat hier die besten Werte, da es eine hohe Wasserlöslichkeit gegenüber einer sehr geringen Dichte hat, was die Anwendung im subgingivalen Bereich, besonders als steriles Pulver, bei geöffnetem Zahnfleisch besonders indiziert. Erfindungsgemäß ist ebenfalls ein Verfahren zur Herstellung eines sterilen Pulvers, insbesondere eines erfindungsgemäßen sterilen Pulvers, vorgesehen, umfassend die Schritte:
- Bereitstellen eines Pulvers und
- Sterilisieren des Pulvers in einem Sterilisierungsvorgangs.

Mittels des erfindungsgemäßen Verfahrens lässt sich mit Vorteil ein steriles Pulver bereitstellen, das sich zur Reinigung von inneren Körperteilen und Implantaten eignet. Alle für das erfindungsgemäße Pulver beschriebenen Merkmale und deren Vorteile lassen sich sinngemäß ebenfalls auf das erfindungsgemäße Verfahren übertragen und andersherum.

Dabei ist unter einem Sterilisierungsvorgang insbesondere ein solcher zu verstehen, nach dessen Durchführung ein Pulver bereitgestellt wird, das sich unmittelbar, d. h. ohne weiteren Behandlungsschritt, als Pulver für ein Pulverstrahlgerät eignet. Hierzu muss der Sterilisierungsvorgang mit Vorteil derart an das Pulver angepasst sein, dass das Pulver nach dem Sterilisierungsvorgang ausreichend trocken sowie feinkörnig ist und sich damit zum Einsatz in einem Pulverstrahlgerät eignet. Vorzugsweise hat sich die Feuchtigkeit im Pulver durch den Sterilisierungsvorgang maximal um 5 %, bevorzugt um maximal 2,5 % und besonders bevorzugt um 1,5 % erhöht. Beispielsweise lässt sich die Feuchtigkeit hierbei mit einem Infrarot-Feuchtemesser feststellen.

Vorzugsweise ist der Sterilisierungsprozess daher angepasst an bzw. abhängig vom Pulver bzw. Pulvertyp. Vorzugsweise ist es vorgesehen, dass das Pulver zeitlich unmittelbar nach dem Sterilisierungsvorgang in eine Pulverkammer für ein Pulverstrahlgerät gefüllt wird. Dadurch lässt sich eine erneute Kontamination des sterilen Pulvers vermeiden. Wahlweise kann das sterile Pulver, falls sich durch den Sterilisationsvorgang Agglomerate gebildet haben, durch einen Wirbel-, Sieb-, Schüttel-, Reib- oder Mühlprozess wieder feinkörnig zerteilt werden.

Gemäß einem weiteren Gesichtspunkt der vorliegenden Erfindung ist es vorgesehen, dass im Sterilisierungsvorgang das Pulver erhitzt wird, insbesondere auf eine Temperatur zwischen 110 °C und 210 °C, bevorzugt auf eine Temperatur unterhalb von 180°C, zwischen 120 °C und 160 °C und besonders bevorzugt auf 160 °C erhitzt wird.

Bei dem Verwenden eine Temperatur von im Wesentlichen 160 °C lässt sich in vorteilhafter Weise in vergleichsweise kurzer Zeit, beispielsweise in zwei Stunden, eine Sterilisierung bewirken, ohne dass das Pulver in seiner Eigenschaft zur Bildung eines Pulver-Luft-Gemischs beeinträchtigt wird. Die Verwendung von niedrigeren Temperaturen gestattet mit Vorteil, einer größeren Zahl an Pulvern bzw. Pulverarten, sterilisiert zu werden, ohne in ihrer Eigenschaft zur Bildung des Pulver-Luft-Gemischs für ein Pulverstrahlgerät beeinträchtigt zu werden.

Zweckmäßig ist es vorgesehen, dass das Pulver zwischen 0,5 und 60 Stunden, bevorzugt zwischen 1 und 40 Stunden, und besonders bevorzugt im Wesentlichen zwei Stunden bei Temperaturen von 100°C bis 250°C, bevorzugt 120°C bis 160°C erhitzt wird. Beim zwei Stunden andauernden Erhitzen lässt sich vergleichsweise schnell eine ausreichende Sterilisierung realisieren, dazu eignen sich Temperaturen von etwa 160°C. Dies wirkt sich insbesondere in Hinblick auf die Herstellungsdauer und somit auch auf die Kosten des sterilen Pulvers positiv aus.

Bei tieferen Temperaturen, wie z.B. 120°C und einem längeren Erhitzen, beispielswiese einem zwischen 20 und 40 Stunden andauernden Erhitzen, lässt sich sicherstellen, dass das Pulver ausreichend sterilisiert ist. Besonders bevorzugt ist es vorgesehen, dass ein Pulver, das Erythritol, Trehalose und/oder Glycin umfasst, zwei Stunden lang bei 160 °C erhitzt wird. Alternativ ist es auch denkbar, dass das Pulver, das Erythritol, Trehalose und/oder Glycin umfasst, bei 120 °C 40 Stunden lang erhitzt wird.

Die Löslichkeiten bzw. Schmelzpunkt ergeben sich wie folgt:

**(Tabelle 2)**

| Pulver | Löslichkeit in Wasser (g/l bei 20°C) | Schmelzpunkt (°C) |
|---|---|---|
| Erythritol | 540 | 121 |
| Trehalose | 690 | 215 |
| Glycin | 225 | 233 |
| Natriumbikarbonat | 96 | 851 |

Erkennbar ist, dass sich die vorbeschriebene Erhitzungssterilisation besonders für Natriumbikarbonat eignet, während die anderen Pulver, insbesondere Erythritol, durch andere Sterilisationsverfahren geeigneter zu sterilisieren sind. Da die Schmelzpunkte dieser und der meisten anderen Pulver nicht weit entfernt von den Behandlungstemperaturen liegen, ist ein Verschmelzen der Pulverteilchen oder ein unerwünschtes Anheften möglich. Auch die Behältnisse müssen temperaturbeständig sein, was zu weiteren Einschränkungen führt.

In einer weiteren Ausführungsform der vorliegenden Erfindung ist es vorgesehen, dass das Pulver im Sterilisierungsvorgang einer Strahlung, insbesondere β-, γ-Röntgenstrahlung oder/oder UV-Strahlung, ausgesetzt wird. Denkbar ist beispielsweise auch ein Beschuss des Pulvers mit einem Elektronenstrahl. Im Falle einer Röntgenstrahlung wird das Pulver vorzugsweise der Strahlung einer radioaktiven Cobalt-60 (⁶⁰CO) oder Caesium-137 (¹³⁷ Cs)- Quelle ausgesetzt. Zur Sterilisation des Pulvers werden beispielsweise β- und/oder γ-Strahlen eingesetzt. Während die β-Strahlen aufgrund ihrer geringen Eindringtiefe nur zur Oberflächensterilisation geeignet sind, besitzen γ -Strahlen eine hohe Durchdringungstiefe.

Eine solche Sterilisations-Bestrahlung, z.B. mit einer radioaktiven Beta- oder Gamma-Bestrahlung, von Pulverteilchen, führt allerdings zu Veränderungen von Geruch und Aussehen, was unerwünscht ist.

Vorstellbar ist es auch, dass das Pulver im Sterilisierungsvorgang einem Dampf, insbesondere Wasserdampf oder Wasserstoffperoxid-Dampf, ausgesetzt wird. Beispielsweise lässt sich das Pulver hierzu in einem Autoklaven erhitzen, wobei ein Innenraum des Autoklaven mit Wasserdampf oder Wasserstoffperoxid-Dampf, insbesondere vollständig mit Wasserdampf oder Wasserstoffperoxid-Dampf, gefüllt ist. Vorzugsweise wird das Pulver auf eine Temperatur zwischen 60 °C und 132 °C im Autoklaven für eine Zeitdauer zwischen 30 min und 60 min erhitzt. Bei einer Dauer von 60 min und einer Temperatur von 132 °C lässt sich eine Mehrheit aller Mikroorganismen zerstören und das Pulver erlangt eine Resistenzstufe VI. Mit Vorteil hat sich herausgestellt, dass sich Kalziumkarbonat unter Verwendung von sterilisierendem Dampf sterilisieren lässt.

Die Wasserlöslichkeit der Pulverteilchen nach einer bevorzugten Ausführungsform der Erfindung bedingt allerdings bestimmte Einschränkungen bei der Sterilisierung, da ein Befeuchten zum Verkleben der Pulverpartikel führen würde. Dampfsterilisation ist daher bei wasserlöslichen Teilchen schwierig.

Gemäß einer bevorzugten Ausführungsform der vorliegenden Erfindung und insbesondere für wasserlösliche und/oder hitzeempfindliche Pulver, hat sich überraschend herausgestellt, dass das Pulver im Sterilisierungsvorgang Ethylenoxid (ETO), insbesondere ein Ethylenoxidgas, ausgesetzt wird. Ethylenoxidgas tötet mit Vorteil Bakterien, Viren und Pilze ab. Die Sterilisation mit ETO ist insbesondere ein Niedrigtemperatur Prozess, der bei vorzugsweise 5 bis 100 C°, bevorzugt 2 bis 80 °C und besonders bevorzugt 37 bis 63 °C durchgeführt wird. Daher kann es zur Begasung von hitzeempfindlichen Substanzen verwendet werden. ETO wird vorzugsweise in gasförmiger Form und beispielsweise mit anderen Substanzen, wie z.B. CO₂ oder Dampf, gemischt. Mit Vorteil eignet sich dieses Verfahren für solche Pulver, die hohen Temperaturen nicht standhalten, beispielsweise einen niedrigen Schmelzpunkt haben. Die Verwendung von ETO gestattet somit auch den Einsatz von Pulvern mit niedrigem Schmelzpunkt. Insbesondere ist es vorgesehen, dass die Verwendung von ETO im Wesentlichen dampffrei oder dampfreduziert erfolgt, d. h. es wird auf Dampf als Zusatz zum ETO-Gas verzichtet oder der Anteil gegenüber dem üblich verwendeten Anteil reduziert. Ferner ist es vorstellbar, dass eine Luftfeuchte beim Sterilisierungsvorgang überwacht wird. Dadurch lässt sich besonders das leichte und sehr gut wasserlösliche Erythritol, das im subgingivalen Bereich die besten Ergebnisse in der Anwendung erzielt, sterilisieren.

Als besonders vorteilhaft hat sich ein Sterilisierungsvorgang mit ETO auch im Falle von Natriumbikarbonat erwiesen, insbesondere von Natriumbikarbonat mit einer Korngröße von im Wesentlichen 17 µm.

In einer weiteren Ausführungsform der vorliegenden Erfindung ist es vorgesehen, dass das Pulver einer Natriumhypochlorit (NaClO) - und/oder einer Chlorhexidin (CHX) -Lösung ausgesetzt wird. Vorzugsweise wird das Pulver nacheinander einer Natriumhypochlorit (NaClO) - und/oder einer Chlorhexidin (CHX) -Lösung ausgesetzt, insbesondere wird eine Chlorhexidin (CHX) -Lösung zeitlich nach der Natriumhypochlorit-Lösung verwendet. Dadurch lässt sich das Pulver sterilisieren. Vorzugsweise wird eine 0,01 bis 6 Gew-% Natriumhypochlorit-Lösung, insbesondere eine 0,1 Gew-% Natriumhypochlorit-Lösung, und/oder eine 0,01 bis 2 Gew-% Chlorhexidin (CHX) -Lösung, insbesondere eine 0,1 Gew-%-Lösung, verwendet. Dabei gehen die Mikroorganismen in Lösung und lassen sich zusammen mit der verbrauchten Lösung entfernen, beispielsweise ausspülen.

Ferner ist es vorstellbar, dass das Pulver und die Natriumhypochlorit (NaClO) - und/oder einer Chlorhexidin (CHX) -Lösung einer mechanischen Behandlung, wie z. B. einer Ultraschallbehandlung unterzogen werden. Beispielsweise wird im Sterilisationsvorgang ein Verfahren aus der Druckschrift US 2015 / 0 352 023 A1 verwendet, auf deren Inhalt bezüglich der Zerstörung bzw. Entfernung von Mikroorganismen hiermit ausdrücklich Bezug genommen wird. Bevorzugt ist es vorgesehen, dass das Pulver vor seiner Nutzung, beispielsweise dem Einfüllen in einer Pulverkammer, ausreichend getrocknet wird.

Da auch bei einer ETO Sterilisation Wasserdampf eingesetzt wird, um die Wirksamkeit der Sterilisation zu erhöhen, ist auch hier mit Einschränkungen zu rechnen. Um dem zu begegnen verwendet die vorliegende Erfindung mit Vorteil einen feuchtigkeitsreduzierten Zyklus. Bei der Ethylenoxid-Sterilisation (ETO) erfolgt die Behandlung in der Regel zwischen 30°C - 60°C, bei einer relativen Luftfeuchtigkeit über 30%, einer Gas-Konzentration von 200 mg/l - 800 mg/l und nimmt einen Zeitraum von mindestens 3 Stunden in Anspruch.

Da das Sterilgut, also das Pulver, in seiner Verpackung sterilisiert wird, müssen die aktiven Substanzen durch diese Verpackung hindurch wirken können. Daher wird die Verpackung normalerweise einer 100% Feuchte ausgesetzt, die dann innerhalb der Verpackung einen Wert von etwa 10% bis 50%, bevorzugt etwa 30% annimmt, was für ein ETO Verfahren einen geeigneten Wert darstellt. Für die Pulver der vorleigenden Erfindung hat sich ein Wert von 85% Feuchte (ausserhalb der Verpackung) als ideal erwiesen, wobei die Grenze bei etwa 90% liegt. Das ETO selbst scheint keinen negativen Einfluss auf die getesteten Pulver zu haben. Als besonders bevorzugte Sterilisationstemperatur hat sich 42°-52 C bewährt, besonders bevorzugt 46-48°C.

Die Pulver der vorliegenden Erfindung sind hygroskopisch. Erfindungsgemäss werden den Pulvern daher amorphe Kieselsäure (Siliziumdioxid), insbesondere in einem Volumenverhältnis von 0,1 bis 2,5 %, beigemischt. Dadurch kann eine Feuchtigkeitsaufnahme der Pulver bei der Sterilisierung verringert, bzw. sogar ganz verhindern werden. Die Beigabe von amorpher Kieselsäure führt zu einer hydrophoben Beschichtung der Pulverteilchen und begegnet so der Feuchtigkeitsaufnahme der Pulverteilchen bei dem Sterilisationsprozess. Bei weniger hygroskopischen Pulvern kann die Beigabe von amorpher Kieselsäure reduziert oder auf die Beigabe kann sogar ganz verzichtet werden. Als hygroskopisch sind folgende Pulversorten einzustufen: Natriumhydrogenkarbonat, Glyzin und Erythritol. Trehalose ist etwas weniger empfindlich.

Für das Gelingen einer Sterilisation ist der Schutz der Pulverteilchen vor Feuchtigkeit, im Hinblick auf den Erhalt der Funktionstüchtigkeit eines wasserlöslichen Pulvers als Strahlmittel, massgeblich.

Eine weitere Möglichkeiten zum Erhalt eines sterilen aber nicht hygroskopischen Pulvers ist die Verwendung von Kalziumkarbonat als Strahlmittel. Dieses Pulver ist nicht wasserlöslich und kann daher besser, auch mittles Dampf- / Hitzesterilisation sterilisiert werden.

Erfindungsgemäß ist ebenfalls eine Verwendung eines erfindungsgemäßen sterilen Pulvers vorgesehen. Alle für das erfindungsgemäße Pulver und Verfahren zur Herstellung des sterilen Pulvers beschriebenen Merkmale und deren Vorteile lassen sich sinngemäß ebenfalls auf das erfindungsgemäße Verfahren übertragen und andersherum. Beispielsweise handelt es sich bei dem Pulverstrahlgerät um ein solches, wie es in der Druckschrift WO 2016 142 272 A1 offenbart ist und auf dessen Beschreibung bezüglich des Pulverstrahlgeräts hiermit ausdrücklich Bezug genommen wird. Weiterhin ist es bevorzugt vorgesehen, dass das Pulver zum Befüllen von austauschbaren Pulverkammern verwendet wird.

Weitere Vorteile und Merkmale ergeben sich aus der nachfolgenden Beschreibung bevorzugter Ausführungsformen des erfindungsgemäßen Gegenstands mit Bezug auf die beigefügten Figuren. Einzelne Merkmale der einzelnen Ausführungsform können dabei im Rahmen der Erfindung miteinander kombiniert werden.

Es zeigt:

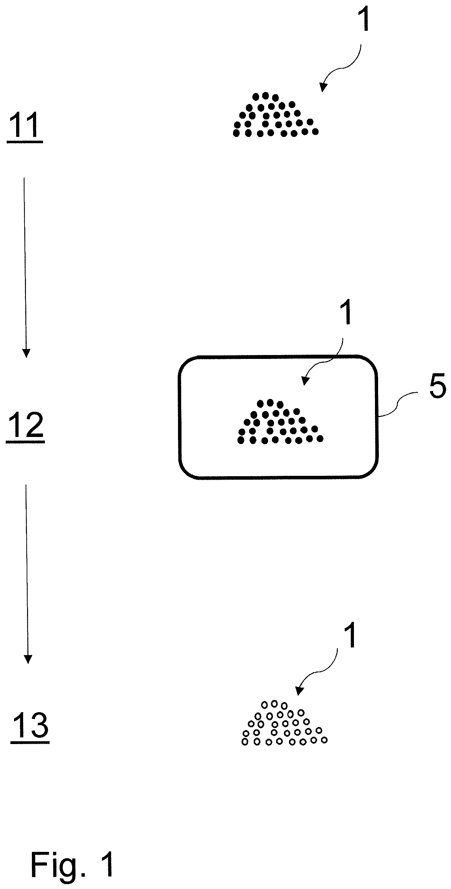

- **Fig.1:**: ein Verfahren zur Herstellung eines sterilen Pulvers gemäß einer beispielhaften Ausführungsform der vorliegenden Erfindung in einem schematischen Flussdiagramm

In **Figur 1** ist ein Verfahren zur Herstellung eines sterilen Pulvers 1 gemäß einer beispielhaften Ausführungsform der vorliegenden Erfindung schematisch in einem Flussdiagramm dargestellt. Insbesondere handelt es sich um ein Pulver 1, das für ein Pulverstrahlgerät vorgesehen ist. Typischerweise wird in solchen Pulverstrahlgeräten ein Pulver-Luft-Gemisch hergestellt, wobei das Pulver-Luft-Gemisch zur Reinigung mittels des Pulverstahlgeräts auf ein zu reinigendes inneres Körperteil bzw. Implantat gerichtet wird und das im Strahl auf das innere Körperteil bzw. Implantat auftreffende Pulver 1 Verunreinigungen bzw. Verschmutzungen abrasiv zu Reinigungszwecken abträgt. Denkbar ist ferner, dass das Pulver-Luft-Gemisch das Pulverstrahlgerät gemeinsam mit einem Fluid verlässt, wobei das Fluid mit dem Pulver-Luft-Gemisch vermischt ist und/oder dieses ummantelt. Mit Vorteil ist es vorgesehen, dass das Pulver 1 zeitlich vor seiner Verwendung im Pulverstrahlgerät außerhalb des Pulverstrahlgeräts in einem Sterilisierungsvorgang 12 sterilisiert wird, d. h. von Mikroorganismen, wie Bakterien und Keimen, befreit wird. Dies gestattet in vorteilhafter Weise die Verwendung eines Pulvers 1 beim Reinigen von inneren Körperteilen bzw. Implantaten, insbesondere ohne dass im Anschluss an das Reinigen eine Sterilisation bzw. ggf. eine erneute Sterilisation des Implantats bzw. des inneren Körperteils erforderlich ist. Insbesondere ist das Pulver 1 derart gestaltet, dass es eine Oberfläche des Implantats bzw. des Inneren Körperteils nicht beschädigt.

Hierzu weist das Pulver 1 vorzugsweise eine Dichte zwischen 0,2 und 3 g/cm³, bevorzugt zwischen 0,2 und 2,4 g/cm³ und besonders bevorzugt zwischen 0,2 und 1,6 g/cm³ auf. Beispielsweise handelt es sich um ein Pulver 1, das Natriumbikarbonat, Kalziumkarbonat, Erythritol, Trehalose und/oder Glycin umfasst, wobei das Pulver, insbesondere ein Natriumbikarbonat umfassendes Pulver, eine durchschnittliche Korngröße zwischen 5 und 100 µm, bevorzugt zwischen 10 und 50 µm und besonders bevorzugt im Wesentlichen von 17 µm aufweist. Hierbei hat sich insbesondere gezeigt, dass sich derartige Pulver 1 auch zur Reinigung von Implantaten eignen, die wegen ihrer hohen Anforderungen an ihre Biokompatibilität und Fähigkeit schnell einzuwachsen, anfällig sind für Oberflächenschädigungen, da die Materialien, aus denen sie gefertigt oder beschichtet sind, porös und/oder hydrophil sind.

In dem in der Figur 1 dargestellten Verfahren wird zunächst ein Pulver 1, insbesondere Natriumbikarbonat mit einer durchschnittlichen Korngröße von 17 µm, bereitgestellt. Dieses Pulver 1 wird anschließend in einer geschlossenen Kammer 5 positioniert, in der der Sterilisierungsvorgang vollzogen wird. Beispielsweise wird das Pulver im Sterilisationsvorgang in der Kammer 5 erwärmt, einer elektromagnetischen Strahlung und/oder einem Dampf ausgesetzt. Als besonders vorteilhaft hat sich herausgestellt, das Pulver 1, insbesondere Natriumbikarbonat mit einer durchschnittlichen Korngröße von 17 µm, einem Ethylenoxid umfassenden Gas auszusetzten. Nach Ablauf einer Verweilzeit des Ethylenoxid umfassenden Gases in der geschlossen Kammer 5 und nach dessen Abzug aus der geschlossen Kammer 5, erfolgt das Bereitstellen 13 des sterilen Pulvers 1. Vorzugsweise wird das Pulver 1 unmittelbar nach dem Sterilisierungsvorgang 12 in eine Pulverkammer für ein Pulverstrahlgerät, insbesondere in eine sterile Pulverkammer, eingefüllt, um eine Dekontamination des sterilen Pulvers 1 unmittelbar nach dessen Herstellung zu vermeiden. Beispielsweise umfasst die geschlossene Kammer 5 einen Ausgang, über den das Pulver 1 unmittelbar in eine Pulverkammer, die beispielsweise kompatibel mit einem Pulverstrahlgerät ist, führbar ist.

### Bezugszeichen:

- 1: Pulver
- 5: Kammer
- 11: Bereitstellen eines Pulvers
- 12: Sterilisationsvorgang
- 13: Bereitstellen steriles Pulver

## Patentansprüche

1. Pulver (1) zur Reinigung eines inneren Körperteils und/oder eines Implantats mittels eines Pulverstrahlgeräts, wobei das Pulver (1) infolge eines Sterilisierungsvorgangs (12) steril ist, **dadurch gekennzeichnet, dass** das Pulver eine Dichte zwischen 0,2 und 1,6 g/cm³ aufweist und dem Pulver amorphe Kieselsäure (Siliziumdioxid) beigemischt ist.

2. Pulver (1) gemäß Anspruch 1, wobei das Pulver (1) Natriumbikarbonat, Kalziumkarbonat, Erythritol, Trehalose und/oder Glycin umfasst.

3. Pulver (1) gemäß einem der vorhergehenden Ansprüche, wobei das Pulver (1), eine durchschnittliche Korngröße zwischen 5 und 100 µm, bevorzugt zwischen 10 und 50 µm und besonders im Wesentlichen von 12-20 µm aufweist.

4. Pulver (1) gemäß einem der vorhergehenden Ansprüche, wobei das Pulver (1) wasserlöslich ist, wobei die Wasserlöslichkeit bei einer Temperatur von 20°C bei über 1 g/l, bevorzugt bei über 50 g/l, bevorzugt bei etwa 100 g/l, oder sogar bei über 100 g/l liegt.

5. Pulver (1) gemäß einem der vorhergehenden Ansprüche, wobei dem Pulver amorphe Kieselsäure (Siliziumdioxid) in einem Volumenverhältnis von 0,1 bis 2,5 % beigemischt ist.

6. Verfahren zur Herstellung eines sterilen Pulvers (1), insbesondere eines sterilen Pulvers (1) gemäß einem der vorhergehenden Ansprüche, umfassend die Schritte:
- Bereitstellen (11) eines Pulvers (1),
- Beimischen von amorpher Kieselsäure (Siliziumdioxid) und
- Sterilisieren des Pulvers (1) in einem Sterilisierungsvorgang (12).

7. Verfahren gemäß Anspruch 6, wobei das Pulver (1) im Sterilisierungsvorgang (12) Ethylenoxid, insbesondere einem Ethylenoxidgas, ausgesetzt wird.

8. Verfahren gemäß Anspruch 6 oder 7, wobei das Pulver einer Natriumhypochlorit (NaClO) - und/oder einer Chlorhexidin (CHX) -Lösung ausgesetzt wird

9. Verfahren gemäß Anspruch 6 bis 8, wobei im Sterilisierungsvorgang (12) das Pulver (1) erhitzt wird, insbesondere auf eine Temperatur zwischen 110 °C und 210 °C, bevorzugt auf eine Temperatur zwischen 120 °C und 160 °C und besonders bevorzugt auf 160 °C erhitzt wird.

10. Verfahren gemäß einem der Ansprüche 6 bis 9, wobei das Pulver (1) im Sterilisierungsvorgang (12) einer Strahlung, insbesondere β-, γ-Röntgenstrahlung oder/oder UV-Strahlung, ausgesetzt wird.

11. Verfahren gemäß einem der Ansprüche 6 bis 10, wobei das Pulver (1) im Sterilisierungsvorgang (12) einem sterilisierenden Dampf ausgesetzt wird.

12. Verwendung eines sterilen Pulvers (1) gemäß einem der Ansprüche 1 bis 5 in einem Pulverstrahlgerät zur Reinigung eines inneren Körperteils oder eines Implantats.

## Claims

1. A powder (1) for cleaning an inner body part and/or an implant by means of a powder jet device, wherein the powder (1) is sterile as a result of a sterilization process (12), **characterized in that** the powder has a density between 0.2 and 1.6 g/cm³, and amorphous silia (silicon dioxide) is admixed to the powder.

2. The powder (1) according to claim 1, wherein the powder (1) comprises sodium bicarbonate, calcium carbonate, erythritol, trehalose and/or glycine.

3. The powder (1) according to one of the preceding claims, wherein the powder (1), has an average particle size between 5 and 100 µm, preferably between 10 and 50 µm and especially substantially from 12-20 µm.

4. The powder (1) according to one of the preceding claims, wherein the powder (1) is water soluble, the water solubility at a temperature of 20°C being above 1 g/l, preferably above 50 g/l, preferably about 100 g/l, or even above 100 g/l.

5. The powder (1) according to one of the preceding claims, wherein amorphous silica (silicon dioxide) is admixed to the powder in a volume ratio of 0.1 to 2.5%.

6. A method for producing a sterile powder (1), especially a sterile powder (1) according to one of the preceding claims, comprising the steps of:
- providing (11) a powder (1),
- admixing amorphous silica (silicon dioxide), and
- sterilizing the powder (1) in a sterilization process (12).

7. The method according to claim 6, wherein the powder (1) is exposed to ethylene oxide, especially an ethylene oxide gas in the sterilization process (12).

8. The method according to claim 6 or 7, wherein the powder is exposed to a sodium hypochlorite (NaClO) solution and/or a chlorhexidine (CHX) solution.

9. The method according to claims 6 to 8, wherein, in the sterilization process (12), the powder (1) is heated, especially to a temperature between 110 °C and 210 °C, preferably to a temperature between 120 °C and 160 °C and particularly preferably to 160 °C.

10. The method according to one of the claims 6 to 9, wherein the powder (1) is exposed to radiation, especially β-, γ-x-ray radiation or/and UV radiation in the sterilization process (12).

11. The method according to one of the claims 6 to 10, wherein the powder (1) is exposed to a sterilizing steam in the sterilization process (12).

12. Use of a sterile powder (1) according to one of claims 1 to 5 in a powder jet device for cleaning an inner body part or an implant.

## Revendications

1. Poudre (1) pour le nettoyage d'une partie interne du corps et/ou d'un implant au moyen d'un appareil à jet de poudre, la poudre (1) étant stérile à la suite d'un processus de stérilisation (12), **caractérisée en ce que** la poudre présente une densité comprise entre 0,2 et 1,6 g/cm³, et de la silice amorphe (dioxyde de silicium) est mélangée à la poudre.

2. Poudre (1) selon la revendication 1, la poudre (1) comprenant du bicarbonate de sodium, du carbonate de calcium, de l'érythritol, du tréhalose et/ou de la glycine.

3. Poudre (1) selon l'une des revendications précédentes, la poudre (1) présentant une granulométrie moyenne comprise entre 5 et 100 µm, de préférence entre 10 et 50 µm et en particulier sensiblement entre 12 et 20 µm.

4. Poudre (1) selon l'une des revendications précédentes, la poudre (1) étant soluble dans l'eau, la solubilité dans l'eau à une température de 20°C étant supérieure à 1 g/l, de préférence supérieure à 50 g/l, de préférence égale à environ 100 g/l, ou même supérieure à 100 g/l.

5. Poudre (1) selon l'une des revendications précédentes, dans laquelle de la silice amorphe (dioxyde de silicium) est mélangée à la poudre dans un rapport volumique de 0,1 à 2,5%.

6. Procédé de préparation d'une poudre stérile (1), en particulier d'une poudre stérile (1) selon l'une des revendications précédentes, comprenant les étapes consistant à :
- fournir (11) une poudre (1),
- ajouter de la silice amorphe (dioxyde de silicium), et
- stériliser la poudre (1) dans un processus de stérilisation (12).

7. Procédé selon la revendication 6, dans lequel, dans le processus de stérilisation (12), la poudre (1) est exposée à l'oxyde d'éthylène, en particulier à un gaz d'oxyde d'éthylène.

8. Procédé selon la revendication 6 ou 7, dans lequel la poudre est exposée à une solution d'hypochlorite de sodium (NaClO) et/ou de chlorhexidine (CHX).

9. Procédé selon les revendications 6 à 8, dans lequel, dans le processus de stérilisation (12), la poudre (1) est chauffée, en particulier à une température comprise entre 110 °C et 210 °C, de préférence à une température comprise entre 120 °C et 160 °C, et de manière particulièrement préférée à 160 °C.

10. Procédé selon l'une des revendications 6 à 9, dans lequel, dans le processus de stérilisation (12), la poudre (1) est exposée à un rayonnement, en particulier à un rayonnement X β, y, ou/et à un rayonnement UV.

11. Procédé selon l'une des revendications 6 à 10, dans lequel, dans le processus de stérilisation (12), la poudre (1) est exposée à une vapeur stérilisante.

12. Utilisation d'une poudre stérile (1) selon l'une des revendications 1 à 5 dans un appareil à jet de poudre pour le nettoyage d'une partie interne du corps ou d'un implant.
